(19) Europäisches Patentamt
European Patent Office
Office européen des brevets

(11) EP 0 626 936 B1

(12) EUROPÄISCHE PATENTSCHRIFT

(45) Veröffentlichungstag und Bekanntmachung des Hinweises auf die Patenterteilung:
**04.12.1996 Patentblatt 1996/49**

(21) Anmeldenummer: **93902050.9**

(22) Anmeldetag: **21.01.1993**

(51) Int Cl.[6]: **C07C 17/395**

(86) Internationale Anmeldenummer:
**PCT/DE93/00061**

(87) Internationale Veröffentlichungsnummer:
**WO 93/16974 (02.09.1993 Gazette 1993/21)**

# (54) VERFAHREN ZUR REINIGUNG VON PERFLUORKOHLENSTOFFEN

METHOD OF PURIFYING PERFLUOROCARBONS

PROCEDE DE PURIFICATION DE PERFLUOROCARBONES

(84) Benannte Vertragsstaaten:
**AT BE CH DE ES FR GB IT LI SE**

(30) Priorität: **21.02.1992 DE 4205341**

(43) Veröffentlichungstag der Anmeldung:
**07.12.1994 Patentblatt 1994/49**

(73) Patentinhaber: **PHARMPUR GmbH**
**86156 Augsburg (DE)**

(72) Erfinder: **MEINERT, Hasso**
**D-7910 Neu-Ulm (DE)**

(74) Vertreter: **Nöth, Heinz, Dipl.-Phys.**
**Patentanwalt,**
**Mozartstrasse 17**
**80336 München (DE)**

(56) Entgegenhaltungen:
**EP-A- 0 151 697**

- **CHEMICAL ABSTRACTS, vol. 106, no. 19, 11. Mai 1987, Columbus, Ohio, US; abstract no. 156293r, HIROSHI OKAZAKI ET AL. 'Purification of nitrogen- containing perfluoro compounds' Seite 673 ;Spalte 2 ; & JP-A-61 251 643 (NIPPON STEEL CHEMICAL) 8 November 1986**
- **JOURNAL OF FLUORINE CHEMISTRY Bd. 51, Nr. 1, Januar 1991, LAUSANNE CH Seiten 53 - 73 H. MEINERT ET AL. "On the electrochemical Fluorination of Derivatives of Morpholine and Piperidine" cited in the application see page 71**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 626 936 B1

Printed by Jouve, 75001 PARIS (FR)

## Beschreibung

Die Erfindung betrifft ein Verfahren zur Reinigung von Perfluorkohlenstoffen bzw. Perfluorkohlenstoffgemischen mit wasserstoffhaltigen und/oder Kohlenstoff-Kohlenstoff-Doppelbindungen enthaltenden Verunreinigungen.

Perfluorkohlenstoffe, die in bekannter Weise, z.B. durch Elektrofluorierung oder einen $CoF_3$-Prozeß oder durch Direktfluorierung hergestellt werden, enthalten in Abhängigkeit vom Herstellungsverfahren wechselnde Mengen an teilfluorierten Substanzen, d.h. Verbindungen, die noch Wasserstoff und/oder Kohlenstoff-Kohlenstoff-Mehrfachbindungen im Molekül enthalten. Diese Nebenprodukte besitzen eine gegenüber den perfluorierten Verbindungen erhöhte chemische Reaktivität und Toxizität. Für die meisten Anwendungsgebiete, besonders im Bereich der Medizin und Mikroelektronik, ist es unerläßlich, diese Verbindungen abzutrennen.

Die bisher beschriebenen Verfahren nutzen dazu die im Vergleich zu den Perfluorkohlenstoffen höhere chemische Reaktivität gegenüber Nucleophilen wie z.B. Alkalien, Aminen und Oxidationsmitteln aus.

So wird nach JP 60-112,724 eine Behandlung mit einer sehr hochkonzentrierten Lösung vorgeschlagen. C. Fukaya et al. (10th Int. Symp. Fluorine Chem., Vancouver, Aug., 1-6, 1982) führen eine kombinierte Behandlung mit Diisobutylamin und Kaliumhydroxid durch, die von einer sauren Wäsche gefolgt wird. Nach US 4,328,376 werden zur Reinigung von perfluorierten Ethern Ammoniak oder primäre oder sekundäre Amine verwendet.

Bei den vorgenannten Verfahren werden die zu reinigenden Produkte über mehrere Tage unter Normaldruck am Rückfluß gekocht. LeBlanc et al. (Nouveau J. Chim. 8 (1984) 251) beschreiben eine Reinigung durch Schütteln mit 10%iger wäßriger KOH und nachfolgender Filtration über Aktivkohle und Aluminiumoxid. Auch Weeks (US 3,696,156) führt die Reinigung mit Natriumhydroxid an $Al_2O_3$ durch.

Conte et al. (Chimicaoggi 11 (1988) 61) beschreiben ein Reinigungsverfahren für Perfluor-N, N-diethylcyclohexylamin mittels Diethylamin im Autoklaven bei 120°C, Waschen des Reaktionsproduktes mit Wasser und nachfolgendem Autoklavieren mit 10%iger alkoholischer KOH-Lösung bei 120°C und 5 atm.

Nicht als Reinigungsverfahren, aber als Verfahren zum chemischen Abbau von H-haltigen Fluorverbindungen wird von Huang et al. (J. Fluorine Chem. 45(1989) 239) die Behandlung mit einem Gemisch von CaO, NaOH und Wasser angeführt. Diese Reaktion geht auf eine Veröffentlichung von Wallin et al. (Anesthesia and Analgesia 54 (1975) 758) über die Beständigkeit eines fluorierten Ethers als Anesthetikum zurück.

Meinert et al. (Forschungsbericht, AdW, Berlin 1986) benutzen zur analytischen Erfassung des eliminierbaren HF und somit der Fluoridionen aus Verbindungen mit $-CF_2CHF$-Gruppierungen deren Umsetzung mit Hexamethylendiamin bei 120°C. Diese Umsetzung wurde später von Groß et al. (Mittbl. Chem. Ges. 37 (1990) 1461) bei Gegenwart von Nonan in homogener Phase durchgeführt.

Nach Radeck et al. (DD 287478) werden die zu reinigenden Perfluorcarbone mit hochvalenten Metallfluoriden bei Temperaturen von 350 - 500°C über einen Zeitraum von 0,5 bis 6 Stunden behandelt.

Meinert et al. (1989 Intern. Chem. Congr. Pacific Basin Soc., Honolulu, Nr. 174; schlußbericht BMFT-Projekt "PFC-Emulsionen, 1988; J. Fluorine Chem. 51 (1991) 53) benutzen eine Druckreinigung, wobei das Rohprodukt mit konz. KOH, sekundärem Amin und CaO bei 170 - 200°C autoklaviert wird.

Von einigen Autoren werden auch extraktive Verfahren zur Reinigung von Perfluorkohlenstoffen vorgeschlagen, so z.B. in US 3,887,629 eine Flüssig-Flüssig-Extraktion mit Kohlenwasserstoffen oder in US 3,449,218 eine azeotrope Destillation von Perfluorkohlenstoffen mit sauerstoffhaltigen Kohlenwasserstoffen, wie z.B. Aceton. Die Reinheitsgrade der Perfluorkohlenstoffe, die damit erreicht werden, sind jedoch für biologische und medizinische Zwecke noch nicht ausreichend, so daß sie nur als Vorreinigung anzusehen sind.

Bei einem eingangs genannten und aus der JP-A-61251643 bekannten Verfahren werden zur Reinigung von stickstoffhaltigen Perfluorkohlenstoffen die zu reinigenden Perfluorkohlenstoffe mit KOH, einem sekundären Amin und Alkoholat-Ionen erhitzt.

Ausgehend hiervon liegt der Erfindung die Aufgabe zugrunde, ein verbessertes Verfahren zur Reinigung von Perfluorkohlenstoffen zu schaffen.

Diese Aufgabe wird erfindungsgemäß durch die im Patentanspruch 1 beanspruchten Merkmale gelöst.

Die zu reinigenden Perfluorkohlenstoffe bzw. Perfluorkohlenstoffgemische werden mit einem sekundären Amin, $R_2NH$, und Alkoholat-Ionen und einer starken Base, insbesondere KOH oder NaOH bei Gegenwart von $Ca^{2+}$ - oder $Ba^{2+}$ -Ionen umgesetzt. Dadurch werden hochreine Perfluorkohlenstoffe erhalten, die absolut frei von eventuell noch gebundenen Wasserstoffatomen oder fluorolefinischen Doppelbindungen sind und unmittelbar in der Medizin, Biologie und Elektronik eingesetzt werden können.

Die so gereinigten Perfluorkohlenstoffe bzw. Perfluorkohlenstoffgemische sind chemisch inerte, biokompatible Verbindungen, Gase oder meist Flüssigkeiten, die aufgrund dieser Eigenschaften in Medizin, Biologie und Mikroelektronik verwendet werden können.

Die zu reinigenden Perfluorkohlenstoffe der Perfluorkohlenstoffgemische sind im wesentlichen Stoffe oder Gemische von Substanzen, deren Moleküle aus einem Kohlenstoffgerüst bestehen, bei dem ein oder mehrere Kohlenstoffatome durch Heteroatome wie Stickstoff- und/oder Sauerstoff- und/oder Schwefel- und/oder Phosphoratomen ersetzt

sein können und in denen ausschließlich Einfachbindungen zwischen den Kohlenstoffatomen bzw. zwischen den Kohlenstoff- und Heteroatomen vorliegen und alle weiteren Bedingungen durch Fluoratome abgesättigt sind.

Sie können aus den entsprechenden CH-analogen Ausgangsverbindungen, insbesondere durch elektrochemische Fluorierung, aber auch durch andere Fluorierungsverfahren hergestellt werden.

Herstellungsbedingt enthalten diese Produkte als Verunreinigungen Verbindungen, die noch Wasserstoffatome und/oder Kohlenstoff-Kohlenstoff-Doppelbindungen aufweisen und die in ihrer Grundstruktur mit den perfluorierten Verbindungen identisch sein können.

Durch die Erfindung wird ein Reinigungsverfahren geschaffen, das in Wirksamkeit und Durchführbarkeit dem bisherigen Stand der Technik überlegen ist.

Flüssige Perfluorkohlenstoffe sind in Wasser praktisch unlöslich und in den meisten organischen Lösungsmitteln nur sehr wenig löslich. Perfluorkohlenstoffe sind nur ineinander löslich oder löslich in Halogenfluorkohlenstoffen.

Werden nach dem Stand der Technik die zu reinigenden Perfluorcarbone mit starken Basen im wäßrigen Milieu und Aminen umgesetzt, stellt dieses Reaktionsgemisch ein System von drei ineinander nicht mischbaren Phasen dar: Perfluorkohlenstoff/Lauge / Amin. Ein Erhitzen am Rückfluß bringt zwar mehr Durchmischung und damit Kontakt der Reaktionspartner, doch erfordert dies ein wochenlanges Kochen. Bei der Umsetzung im Autoklaven werden die Reaktionsverhältnisse bezüglich der Durchmischung und der Reaktionszeit verbessert.

Eine Umsetzung des zu reinigenden Produktes mit Amin ohne starke, wäßrige Basen jedoch schließt die Entfernung des eliminierbaren HF unter Neutralisation im wäßrigen Mileu mittels starker Basen aus. Auch wenn allein Amine als Reaktionsmedium eingesetzt werden, fallen bereits bei halbtechnischen Aufarbeitungen viel zu große Mengen an umweltbelastenden Aminen an.

Reinigungsverfahren, die über mehrere Reaktionsschritte gehen, wobei zunächst mit Aminen Fluorolefine gebildet werden, die dann abgetrennt, gewaschen und nachfolgend mit Alkoholaten in alkoholischer Lösung umgesetzt werden, sind sehr aufwendig und verbessern die vorgenannten Reinigungseffekte nicht wesentlich.

Verfahren der Nachfluorierung mittels fluor aktiven Verbindungen, z.B. hochvalenten Metallfluoriden, bei Temperaturen von 400 - 500° C stellt ein Nachfluorierungsverfahren, insbesondere für sehr unvollständig fluorierte Verbindungen dar. Bei den genannten Temperaturen finden aber bereits deutliche Bindungsspaltungen und Zersetzungen der Ausgangsprodukte statt, was einer Hochreinigung entgegensteht.

Das Reinigungsverfahren nach der Erfindung kann sich dahingehend auszeichnen, daß alle Reaktionsschritte in einer homogenen Phase dadurch erreicht werden, daß die ineinander nicht oder kaum mischbaren Phasen durch die Anwendung eines wirksamen Tensides zumindest partiell und ausreichend vereinheitlicht werden. Dabei findet in der starken Lauge die Eliminierung von noch gebundenem Wasserstoff als HF statt. Die daraus gebildeten Fluoridionen werden als schwerlösliches $CaF_2$ aus dem Reaktionsgleichgewicht mittels des gleichzeitig eingesetzten CaO bzw: Ca $(OH)_2$ entfernt. Danach erfolgt die nukleophile Addition an fluorolefinische Doppelbindungen von sowohl Alkoholat ($RO^-$), vorzugsweise gebildet aus einem einzusetzenden einwertigen Alkohol (ROH mit R = $CH_3$, $C_2H_5$, $C_3H_7$, $(CH_3)_2$ CH u.a.) im stark alkalischem Medium als auch nach abermaliger HF-Eliminierung abschließend von sekundärem Amin statt. Bevorzugt in einer einheitlichen Phase sind alle Reaktanten voll umsetzbar, wobei gemäß der Beteiligung ionischer Spezies am gesamten Umsetzungsprozeß ein wäßriges, d.h. differenzierendes Medium mitbeteiligt ist.

Anstelle von CaO ist auch BaO bzw. $Ba(OH_2)$ einsetzbar, weil auch hierbei schwerlösliches $BaF_2$ gebildet wird und im nachfolgenden Aufarbeitungsprozeß bei der Behandlung mit verd. Schwefelsäure alle $Ba^{2+}$-Ionen quantitativ als $BaSO_4$ gefällt werden.

Die Reaktionen können durch folgende Gleichungen beschrieben werden:

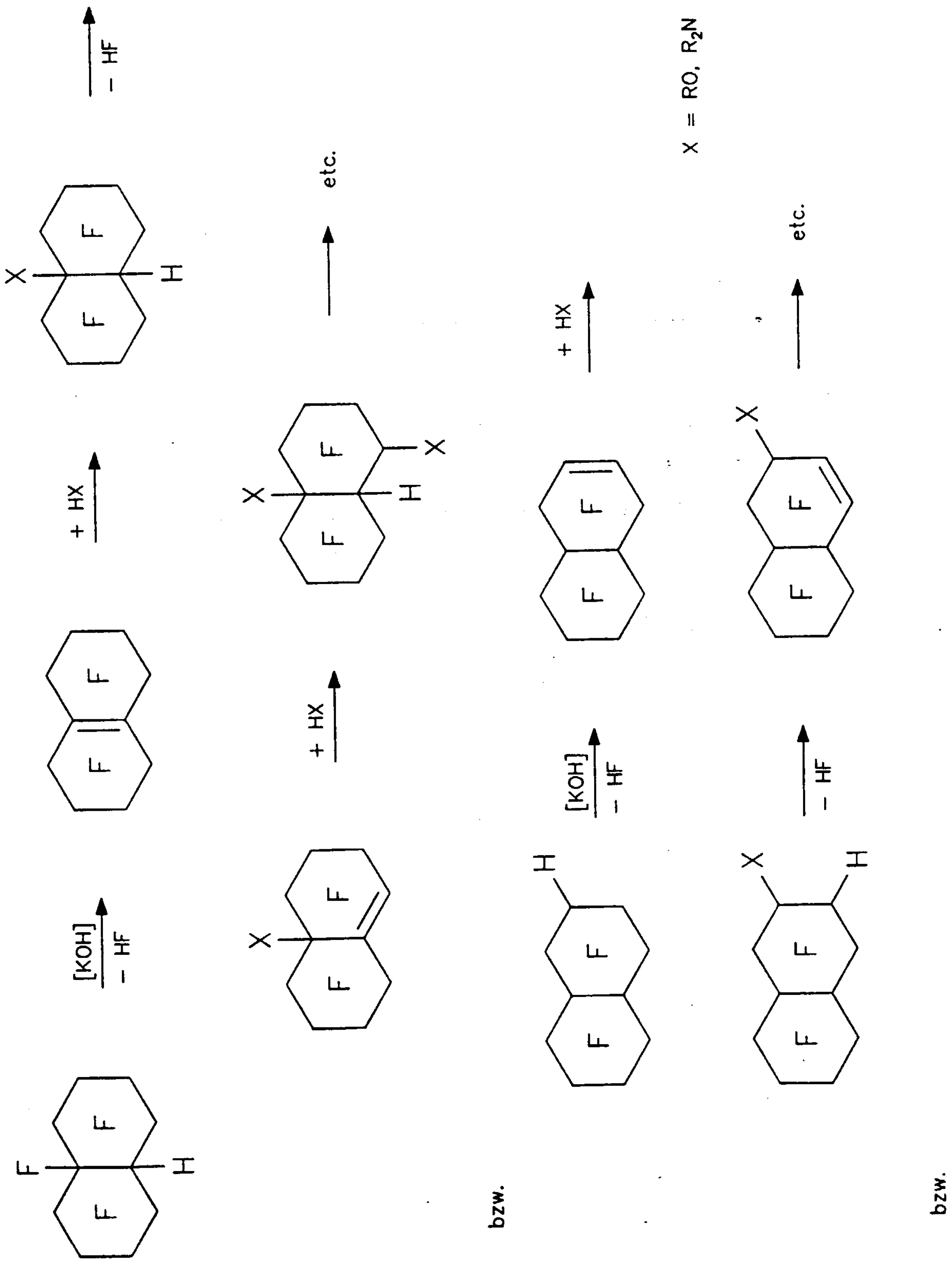

[KOH]
- HF
+ HX
- HF
+ HX
etc.
bzw.
[KOH]
- HF
+ HX
- HF
etc.
bzw.
X = RO, R2N

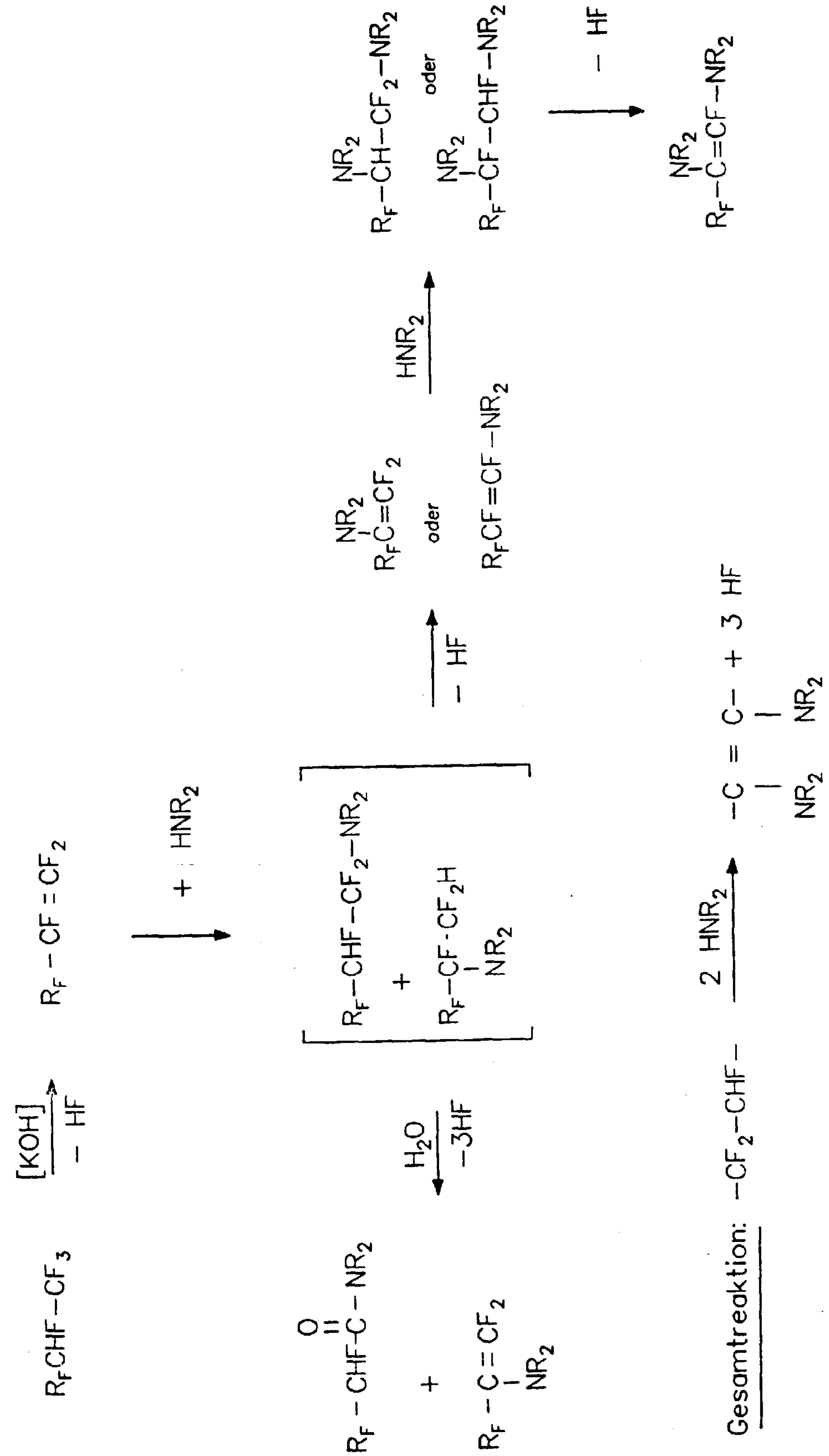
[KOH]
− HF
+ HNR2
H2O
−3HF
− HF
oder
HNR2
oder
− HF
Gesamtreaktion:
2 HNR2
+ 3 HF

Das zu verwendende Tensid kann ein ionisches oder nichtionisches Tensid sein. Es ist gegenüber Laugen und Aminen sowie bei den zur Umsetzung vorgeschlagenen Temperaturen beständig, und es besitzt Emulgiereigenschaften gegenüber den Perfluorkohlenstoffen. Die Tensid-Konzentration braucht nicht höher als 1 % (w/v) zu sein, vorzugsweise beträgt sie ca. 0,05 % bis 0,5 % (w/v).

Bei der Autoklavierung treten in Abhängigkeit von den einzusetzenden Produkten und der erhöhten Temperatur von vorzugsweise 150 - 200° C Drücke von 5 - 20 bar auf. Dadurch wird die Bildung einer homogenen Phase bei bevorzugter Gegenwart des Tensides besonders gefördert.

Es wird aber durch die Tensidwirkung auch schon beim Kochen des Reaktionsgemisches durch mehr Rückfluß bei Normaldruck eine den Ablauf der Reaktion fördernde, partielle und ausreichende Homogenisierung erreicht. Wegen der Schwerlöslichkeit von Perfluorkohlenstoffen in Wasser sollte das Tensid den Anforderungen für die Erstellung von Perfluorkohlenstoff-Emulsionen genügen (u.a. EP 0 231 091; US 3,828,085; US 4,917,930; CH 665 952; DE 2 224 182 C2; DE 2 555 408 C2; DE 3 326 901 C2; US 4,325,972; US 3,962,439; EP 0 261 802; EP 0 282 948; EP 0 282 949; US 3.778,381; US 4,865,836; WO 89/10118), vorausgesetzt, daß das Tensid unter den vorgeschlagenen Prozeßbedingungen beständig ist.

Vorzugsweise eignen sich zur Homogenisierung Tenside aus der Gruppe der im stark alkalischen Milieu beständigen Fluorosurfactants der Typen $R_FCOOH$ oder $R_FSO_3H$ bzw. deren Anionen $R_FCOO^-$ oder $R_FSO_3^-$ ($R_F = CF_3(CF_2)_n$, n = 3 bis 10).

Die Konzentrationen der Tenside betragen maximal 1,0, bevorzugt 0,05 bis 0,5 Gewichtsprozent.

Nach erfolgtem Reinigungsprozeß entmischen sich die Phasen von selbst. Dabei bilden sich zurück die PFC-Phase mit einem geringfügig gelösten Anteil an Amin (<1 %) als die spezifisch schwerste Phase, darüber die wäßrige Lauge mit gelöstem Alkoholat/Alkohol und Tensid, darüber das Amin.

Nach Abtrennen fester Produkte, hauptsächlich Erdalkalifluorid, mittels Filtration werden die flüssigen Phasen getrennt (z.B. mittels Scheidetrichter). Die Perfluorkohlenstoff-Flüssigkeit wird mehrmals mit 2n Säure (z.B. Schwefelsäure oder Salzsäure), 1n $NaHCO_3$-Lösung sowie destilliertem Wasser ausgeschüttelt, über einem wirksamen Trokkenmittel (z.B. wasserfreiem $Na_2SO_4$) getrocknet und abschließend an einer wirksamen Kolonne fraktioniert destilliert.

Die so behandelten Perfluorkohlenstoffe sind gemäß IR-, 1H-NMR-, GC-/MS-Spectroskopie frei von wasserstoffhaltigen und fluorolefinischen Verbindungen. Im Gegensatz zu den Rohprodukten zeigen die gereinigten Perfluorkohlenstoffe als 100 %ig perfluorierte Verbindungen gemäß Untersuchungen an HeLa- oder Molt4- oder $HEP_2$-Zellkulturen keine Hemmung des Zellwachstums. Die Proliferationsraten werden über DNS-Synthese und Proteinbildung ermittelt.

Die erfindungsgemäß hochgereinigten Perfluorkohlenstoffe sind somit für medizinische und biologische Zwecke sowie für die Mikroelektronik direkt einsetzbar.

**Ausführungsbeispiele**

Beispiel 1

1000 ml eines Rohproduktes von Perfluordekalin, das üblicherweise nach dem $CoF_3$-Prozeß gewonnen wurde, werden mit 80 ml Diisobutylamin, 80 ml Ethanol, 80 ml 8n KOH, 5 g CaO und 0,5 g Perfluoroctansäure bei 150 bis 170°C unter Rühren mit 800 Umdrehungen/Minute 72 Standen lang autoklaviert.

Danach wird das umgesetzte Produkt filtriert und die untere Phase des Filtrats im Scheidetrichter abgetrennt. Diese Phase wird zweimal mit 1000 ml 2n Salzsäure, zweimal mit 1000 ml $NaHCO_3$-Lösung und zweimal mit 1000 ml destilliertem Wasser gewaschen.

Danach wird über wasserfreiem Natriumsulfat getrocknet, filtriert und an einer Spaltrohrkolonne (ca. 100 theoretische Böden, Rücklaufverhältnis 10:1 (10 Sekunden Rücklauf, 1 Sekunde Abnahme) bei 141 bis 142° C fraktioniert destilliert.

Im gereinigten Perfluordekalin waren FT-IR-, 1H-NHR-, GC/MS-spektroskopisch keine wasserstoffhaltigen Verbindungen oder Verbindungen mit Doppelbindungen nachweisbar.

Im Vergleich und im Gegensatz zum Rohprodukt zeigt das gereinigte Perfluordekalin keine Proliferationshemmungen bezüglich DNS- und Protein-Synthese an HeLa- oder Molt4- oder $HEP_2$-Zellen.

Als quantitatives Verfahren zur Bestimmung von restlichen CH-Bindungen in Fluorkohlenstoffen eignet sich die Bestimmung von ionisierbarem Fluorid bei der Reaktion des Fluorkohlenstoffs mit Hexamethylendiamin in Nonan bei 120° C gemäß der oben angegebenen Gleichung der Gesamtreaktion. Nach dem Reinigungsverfahren waren demzufolge keine Fluoridionen mehr nachweisbar (Erfassungsgrenze für die Fluoridkonzentration $\leq 10^{-5}$ mol · $1^{-1}$).

Beispiel 2

2500 ml eines Rohproduktes von Perfluoroctan, das üblicherweise nach dem $CoF_3$-Prozeß oder durch Direktfluorierung gewonnen wurde, werden mit 100 ml Diisobutylamin, 100 ml Isopropanol, 200 ml 5n KOH, 15 g BaO und 1 g

Perfluoroctansulfonylfluorid bei 150 bis 170°C unter Rühren (ca. 500 Umdrehungen/Minute) 72 Stunden lang bei ca. 10 bar autoklaviert.

Danach wird das umgesetzte Produkt filtriert und die untere Phase des Filtrats im Scheidetrichter abgetrennt. Diese Phase wird zweimal mit je 400 ml 2n Schwefelsäure, zweimal mit je 400 ml gesättigter $NaHCO_3$-Lösung und zweimal mit je 400 ml zweifach destilliertem wasser gewaschen. Danach wird über wasserfreiem Natriumsulfat getrocknet, filtriert und an einer Spaltrohrkolonne (ca. 100 theoretische Böden, Rücklaufverhältnis 10:1 (10 Sekunden Rücklauf, 1 Sekunde Abnahme) bei 100,5 bis 102,5°C fraktioniert destilliert.

Im gereinigten Perfluoroctan sind gemäß den im Beispiel 1 angegebenen analytischen und zelltoxikologischen Befunden keine CH-haltigen Verbindungen oder-Verbindungen mit Doppelbindungen nachweisbar.

Beispiel 3

Perfluortributylamin, das üblicherweise durch Elektrofluorierung des CH-analogen Ausgangsproduktes gewonnen wird, kann durch den Herstellungsprozeß bedingt eine N-F-Bindung enthalten. Deshalb wird das Rohprodukt einer Vorreinigung durch Umsetzung mit Kaliumiodid unterzogen gemäß:

$$R_F-CF_2-\underset{\substack{|\\ F}}{N}-CF_2-R_F + 2I^- + 4H_2O \rightarrow 2R_FCOOH + I_2 + NH_4F + 2HF + 2F^-$$

2500 ml von vorgereinigtem Perfluortributylamin werden mit 100 ml Diisobutylamin, 100 ml Ethanol, 200 ml 5n KOH, 13 g CaO und 1 g Perfluoroctansäure bei 150 bis 170° C unter Rühren 72 Stunden lang autoklaviert.

Die Aufarbeitung des umgesetzten Produktes erfolgt wie in den Beispielen 1 und 2 angegeben, wobei an der Spaltrohrkolonne bei 178 - 179,5 °C fraktioniert destilliert wird.

Entsprechend den in den Beispielen 1 und 2 angegebenen analytischen und zelltoxikologischen Befunden ist das gereinigte Perfluortributylamin frei von wasserstoffhaltigen Verbindungen oder Verbindungen mit Doppelbindungen. Es ist auch völlig frei von N-F-Bindungen.

**Patentansprüche**

1. Verfahren zur Reinigung von Perfluorkohlenstoffen bzw. Perfluorkohlenstoffgemischen mit wasserstoffhaltigen und/oder Kohlenstoff-Kohlenstoff-Doppelbindungen enthaltenden Verunreinigungen, wobei die zu reinigenden Perfluorkohlenstoffe bzw. Perfluorkohlenstoffgemische mit starken wäßrigen Basen, bei Gegenwart von Alkoholat-Ionen sowie von sekundären Aminen umgesetzt werden, dadurch gekennzeichnet, daß man die Umsetzung in Gegenwart von $Ca^{2+}$ - oder $Ba^{2+}$ -Ionen durchführt.

2. Verfahren nach Anspruch 1, dadurch gekennzeichnet, daß die zu reinigenden Perfluorkohlenstoffe mit den Reaktanten in homogener Phase umgesetzt werden.

3. Verfahren nach Anspruch 1 bis 2, dadurch gekennzeichnet, daß das Reaktionsgemisch, bestehend aus den Phasen Perfluorkohlenstoff/wäßrige Lauge und Alkoholat/sekundäres Amin, mittels eines Tensids homogenisiert wird.

4. Verfahren nach einem der Ansprüche 1 bis 3, dadurch gekennzeichnet, daß das Reaktionsgemisch bei Temperaturen mindestens über dem Siedepunkt des zu reinigenden Perfluorkohlenstoffs autoklaviert wird.

5. Verfahren nach Anspruch 4, dadurch gekennzeichnet, daß das Reaktionsgemisch bei Temperaturen von 150 bis 200°C autoklaviert.

6. Verfahren nach einem der Ansprüche 1 bis 5, dadurch gekennzeichnet, daß das Reaktionsgemisch bei Drücken von 5 bis 20 bar autoklaviert wird.

7. Verfahren nach einem der Ansprüche 1 bis 3, dadurch gekennzeichnet, daß das Reaktionsgemisch unter Normaldruck am Rückfluß gekocht wird.

8. Verfahren nach einem der Ansprüche 1 bis 7, dadurch gekennzeichnet, daß aus unvollständig fluorierten, H-haltigen Verunreinigungen HF durch starke Laugen eliminiert wird.

9. Verfahren nach Anspruch 8, dadurch gekennzeichnet, daß die HF-Eliminierung in Gegenwart von CaO oder BaO bzw. $Ca(OH)_2$ oder $Ba(OH)_2$ unter Bildung von schwerlöslichem $CaF_2$ oder $BaF_2$ durchgeführt wird.

10. Verfahren nach einem der Ansprüche 1 bis 9, dadurch gekennzeichnet, daß durch Zugabe von einwertigem Alkohol zur starken Lauge Alkoholat-Ionen gebildet werden, die als starke Nukleophile mit den durch HF-Eliminierung gebildeten Fluorolefinen weiterreagieren.

11. Verfahren nach Anspruch 10, dadurch gekennzeichnet, daß als einwertiger Alkohol ROH mit R = $CH_3$, $C_2H_5$, $C_3H_7$, $(CH_3)_2CH$ verwendet wird.

12. Verfahren nach einem der Ansprüche 1 bis 11, dadurch gekennzeichnet, daß zur Homogenisierung des Reaktionsgemisches Tenside, welche bei den vorkommenden Prozeßbedingungen beständig sind, verwendet werden.

13. Verfahren nach Anspruch 12, dadurch gekennzeichnet, daß als Tensid ein bei der Herstellung von Perfluorkohlenstoff-Emulsionen bekanntes Tensid verwendet wird.

14. Verfahren nach einem der Ansprüche 1 bis 13, dadurch gekennzeichnet, daß Tenside aus der Gruppe der im stark alkalischen Milieu beständigen Fluorosurfactants der Typen $R_FCOOH$ oder $R_FSO_3H$ ($R_F$ = $CF_3(CF_2)n$, n = 3 bis 10) oder deren Anionen $R_FCOO$ oder $R_FSO_3^-$ verwendet werden.

15. Verfahren nach Anspruch 14, dadurch gekennzeichnet, daß die Tenside in Konzentration von 0,05 bis 0,5, max. 1,0 Gewichtsprozent verwendet werden.

16. Verfahren nach einem der Ansprüche 1 bis 15, dadurch gekennzeichnet, daß nach Beendigung des gesamten Reinigungsprozesses die Tensid-haltige, wäßrige, alkalische Phase wiederverwendet wird.

17. Verfahren nach einem der Ansprüche 1 bis 16, dadurch gekennzeichnet, daß die zu reinigenden Perfluorkohlenstoffe mit den Reaktanten in einem "Eintopf"-Verfahren umgesetzt werden.

18. Verfahren nach einem der Ansprüche 1 bis 17, dadurch gekennzeichnet, daß die zu reinigenden Perfluorkohlenstoffe Verbindungen sind, deren Gerüst aus Kohlenstoffatomen besteht, wobei einzelne Kohlenstoffatome durch Sauerstoff und/oder Stickstoff und/oder Phosphor und/oder Schwefel ersetzt werden können und die darüber hinaus ausschließlich Fluoratome enthalten.

**Claims**

1. A method of purifying perfluorocarbons or perfluorocarbon mixtures with impurities which are hydrogen-bearing and/or which contain carbon-carbon double bonds, wherein the perfluorocarbons or perfluorocarbon mixtures to be purified are reacted with strong aqueous bases in the presence of alcoholate ions and secondary amines, characterised in that the reaction is conducted in the presence of $Ca^{2+}$- or $Ba^{2+}$-ions.

2. A method according to claim 1 characterised in that the perfluorocarbons to be purified are reacted with the reactants in homogenous phase.

3. A method according to claims 1 to 2 characterised in that the reaction mixture comprising the phases perfluorocarbon/aqueous lye and alcoholate/secondary amine is homogenised by means of a tenside.

4. A method according to any one of claims 1 to 3 characterised in that the reaction mixture is autoclaved at temperatures at least above the boiling point of the perfluorocarbon to be purified.

5. A method according to claim 4 characterised in that the reaction mixture is autoclaved at temperatures of from 150 to 200°C.

6. A method according to one of claims 1 to 5 characterised in that the reaction mixture is autoclaved at pressures

of from 5 to 20 bars.

**7.** A method according to one of claims 1 to 3 characterised in that the reaction mixture is boiled under reflux under normal pressure.

**8.** A method according to one of claims 1 to 7 characterised in that HF is eliminated by strong lyes from incompletely fluorinated, H-bearing impurities.

**9.** A method according to claim 8 characterised in that the HF-elimination operation is conducted in the presence of CaO or BaO or $Ca(OH)_2$ or $Ba(OH)_2$, with the formation of $CaF_2$ or $BaF_2$ which is difficult to dissolve.

**10.** A method according to one of claims 1 to 9 characterised in that alcoholate ions are formed by the addition of monohydric alcohol to the strong lye, the alcoholate ions further reacting as strong nucleophilics with the fluoroolefins formed by HF-elimination.

**11.** A method according to claim 10 characterised in that ROH with R = $CH_3$, $C_2H_5$, $C_3H_7$, and $(CH_3)_2CH$ is used as the monohydric alcohol.

**12.** A method according to one of claims 1 to 11 characterised in that tensides which are resistant at the process conditions occurring are used for homogenisation of the reaction mixture.

**13.** A method according to claim 12 characterised in that a tenside which is known in relation to the production of perfluorocarbon emulsions is used as the tenside.

**14.** A method according to one of claims 1 to 13 characterised in that tensides of the group of the fluorosurfactants, which are resistant in a strongly alkaline medium, of the types $R_FCOOH$ or $R_FSO_3H$ ($R_F = CF_3(CF_2)n$, n = 3 to 10) or their anions $R_FCOO$ or $R_FSO_3^-$ are used.

**15.** A method according to claim 14 characterised in that the tensides are used in a concentration of from 0.05 to 0.5, a maximum of 1.0, percent by weight.

**16.** A method according to one of claims 1 to 15 characterised in that after termination of the entire purification procedure the tenside-bearing, aqueous, alkaline phase is re-used.

**17.** A method according to one of claims 1 to 16 characterised in that the perfluorocarbons to be purified are reacted with the reactants in a "one-pot" process.

**18.** A method according to one of claims 1 to 17 characterised in that the perfluorocarbons to be purified are compounds whose lattice structure comprises carbon atoms, wherein individual carbon atoms can be replaced by oxygen and/or nitrogen and/or phosphorus and/or sulphur and which in addition contain exclusively fluorine atoms.

**Revendications**

**1.** Procédé de purification de perfluorocarbures ou de mélanges de perfluorocarbures ayant des impuretés contenant de l'hydrogène et/ou contenant des doubles liaisons carbone-carbone, dans lequel on fait réagir les perfluorocarbures ou les mélanges de perfluorocarbures à purifier avec des bases aqueuses fortes en présence d'ions alcoolate et d'amines secondaires, caractérisé par le fait qu'on effectue la réaction en présence d'ions $Ca^{2+}$ ou $Ba^{2+}$.

**2.** Procédé selon la revendication 1, caractérisé par le fait qu'on fait réagir les perfluorocarbures à purifier avec les réactifs en phase homogène.

**3.** Procédé selon l'une des revendications 1 et 2, caractérisé par le fait qu'on homogénéise le mélange réactionnel, constitué des phases perfluorocarbure/lessive aqueuse et alcoolate/amine secondaire, au moyen d'un agent tensioactif.

**4.** Procédé selon l'une des revendications 1 à 3, caractérisé par le fait qu'on traite le mélange réactionnel à l'autoclave à des températures au moins supérieures au point d'ébullition du perfluorocarbure à purifier.

**5.** Procédé selon la revendication 4, caractérisé par le fait qu'on traite le mélange réactionnel à l'autoclave à des températures de 150 à 200 °C.

**6.** Procédé selon l'une des revendications 1 à 5, caractérisé par le fait qu'on traite le mélange réactionnel à l'autoclave à des pressions de 5 à 20 bar.

**7.** Procédé selon l'une des revendications 1 à 3, caractérisé par le fait qu'on cuit le mélange réactionnel à reflux à la pression normale.

**8.** Procédé selon l'une des revendications 1 à 7, caractérisé par le fait qu'on élimine HF d'impuretés incomplètement fluorées contenant de l'hydrogène au moyen de lessives fortes.

**9.** Procédé selon la revendication 8, caractérisé par le fait qu'on effectue l'élimination de HF en présence de CaO ou BaO ou $Ca(OH)_2$ ou $Ba(OH)_2$ en formant du $CaF_2$ ou du $BaF_2$ difficilement soluble.

**10.** Procédé selon l'une des revendications 1 à 9, caractérisé par le fait qu'on forme par addition d'alcool monovalent à la lessive forte des ions alcoolate qui réagissent comme nucléophiles forts avec les fluoroléfines formées par élimination de HF.

**11.** Procédé selon la revendication 10, caractérisé par le fait qu'on utilise un alcool monovalent ROH avec R = $CH_3$, $C_2H_5$, $C_3H_7$, $(CH_3)_2CH$.

**12.** Procédé selon l'une des revendications 1 à 11, caractérisé par le fait qu'on utilise pour l'homogénéisation du mélange réactionnel des agents tensioactifs stables dans les conditions du processus.

**13.** Procédé selon la revendication 12, caractérisé par le fait qu'on utilise un agent tensioactif connu pour la production d'émulsions de perfluorocarbures.

**14.** Procédé selon l'une des revendications 1 à 13, caractérisé par le fait qu'on utilise des agents tensioactifs du groupe des agents de surface fluorés stables en milieu fortement alcalin des types $R_FCOOH$ et $R_FSO_3H$ (RF = $CF_3(CF_2)$ n, n = 3 à 10) ou leurs anions $R_FCOO^-$ et $R_FSO_3^-$.

**15.** Procédé selon la revendication 14, caractérisé par le fait qu'on utilise les agents tensioactifs en concentration de 0,05 à 0,5, d'au plus 1,0, pour cent en poids.

**16.** Procédé selon l'une des revendications 1 à 15, caractérisé par le fait qu'à l'issue de tout le processus de purification, on réutilise la phase aqueuse alcaline contenant des agents tensioactifs.

**17.** Procédé selon l'une des revendications 1 à 16, caractérisé par le fait qu'on fait réagir les perfluorocarbures à purifier avec les réactifs dans un procédé "à opération unique".

**18.** Procédé selon l'une des revendications 1 à 17, caractérisé par le fait que les perfluorocarbures à purifier sont des composés dont le squelette est constitué d'atomes de carbone, des atomes individuels de carbone pouvant être remplacés par de l'oxygène et/ou de l'azote et/ou du phosphore et/ou du soufre, et qui contiennent en plus uniquement des atomes de fluor.